# EUROPEAN PATENT APPLICATION

(11) **EP 3 354 262 A1**
(43) Date of publication of application: **01.08.2018**
(21) Application number: 18154074.1
(22) Date of filing: 30.01.2018
(51) Int. Cl.: A61K 9/28, A61K 31/4439, A61P 1/04

(54) **ENTERIC COATED PHARMACEUTICAL COMPOSITIONS OF DEXLANSOPRAZOLE**

(30) Priority: 31.01.2017 TR 201701461
(71) Applicant: Sanovel Ilac Sanayi ve Ticaret A.S., 34460 Istanbul (TR)
(72) Inventor: TÜRKYILMAZ, Ali, 34460 Istanbul (TR); ZENGINER, Sibel, 34460 Istanbul (TR); ULUSOY BOZYEL, Müge, 34460 Istanbul (TR); ÖZTÜRK, Erkin, 34460 Istanbul (TR); TASKIN, Abdullah, 34460 Istanbul (TR)
(74) Representative: Sevinç, Erkan

(57) **Abstract**

The present invention is related to enteric coated pharmaceutical compositions comprising dexlansoprazole or pharmaceutically acceptable salts or hydrated forms thereof.

## Description

### Technical Field

The present invention is related to enteric coated pharmaceutical compositions comprising dexlansoprazole or pharmaceutically acceptable salts or hydrated forms thereof.

### Background of the Invention

The active ingredient, dexlansoprazole is the R-enantiomer of lansoprazole which inhibits gastric acid secretion (a proton pump inhibitor that suppresses gastric acid secretion by specific inhibition of the (H+, K+)-ATPase in the gastric parietal cells). Its chemical name is (+)-2-[(*R*)-{[3-methyl-4-(2,2,2-trifluoroethoxy)pyridin-2-yl] methyl} sulfinyl]-1*H-*benzimidazole and its chemical structure is shown in the Formula I.

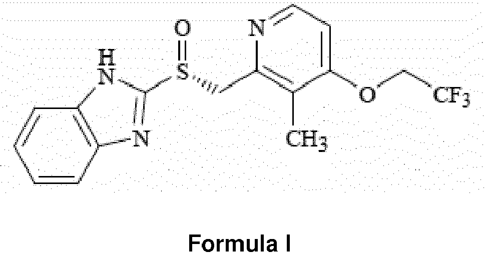

Delayed release capsule form of dexlansoprazole which is indicated for healing of all grades of erosive esophagitis (EE), maintenance of healed EE and relief of heartburn and for the treatment of heartburn associated with non-erosive gastroesophageal reflux disease (GERD) is in the market and it is administered orally in a therapeutic dose of 30 mg and 60 mg.

As other benzimidazole compounds, dexlansoprazole has also poor stability and is unstable to acidic medium, humidty, light and sensitive to heating. When orally administrated, it may not be able to sufficient activity since it is decomposed by gastric acid and the like. Thus, several problems occur in formulating such compound into oral pharmaceutical dosage forms because of the acidic environmental of the stomach. In particular, it will be rapidly decomposed and change colour under moist conditions or in acidic to neutral aqueous solution.

When these compounds are formulated into pharmaceutical preparations for oral administration, they require special techniques to avoid contact of drug with gastric acid of the stomach. One technique most commonly used is to coat these compounds, or its granules or pellets, with an enteric coating. However, the material used in enteric coatings itself is acidic, which can cause the decomposition of the compound. Such decomposition occurs even during the enteric coating process, which results in the coloration of the surface of the drug-containing core. An inert layer is required between the core and the enteric coating to avoid these problems.

Another thing which is known from prior art for improving stability is adding an alkaline stabilizer to the formulation.

In prior art, there are many patents including benzimidazoles such as lansoprazole and its R-enantiomer, dexlansoprazole in several different pharmaceutical compositions. Crystal form of R-lansoprazole is described in EP1129088.

EP0629398 describes a dosage form comprising a drug and an organic acid in a core surrounded by a film that controls the start of release, and further covered by an enteric coating layer. This dosage form is not suitable for substances that are sensitive to acidic degradation as the core comprises an organic acid.

However, no prior art stated that the weight ratio of the separating layer to the stabilizer is efficient for achieving enhanced stability and shelf-life.

Thus, there is still a need for developing pharmaceutical formulations of dexlansoprazole wherein a good stability is achieved in a technologically simple way and having improved manufacturing process which overcomes the above described problems and provides a bioavailable pharmaceutical composition according to the formulations currently used.

Further advantages and embodiments of the present invention will become apparent from the following description.

### Description of the invention

The main object of the present invention is to provide stable enteric coated pharmaceutical compositions comprising dexlansoprazole or pharmaceutically acceptable salts or hydrated forms thereof having enhanced shelf-life with safe and effective dissolution profile and an easy process.

The pharmaceutical formulation of this invention advantageously provides an enteric coated dosage form which is bioequivalent to the capsule dosage form of the same or substantially similar strength.

It has been surprisingly found that using separating layer and the stabilizer in a specific amount by weight, increases the stability of the composition substantially.

In this invention, enteric coated pharmaceutical compositions comprise;
a. a core comprising dexlansoprazole or pharmaceutically acceptable salts or hydrated forms thereof, one or more stabilizer and at least one pharmaceutically acceptable excipient
b. a separating layer comprising one or more coating agents,
c. an enteric coating layer comprising at least one enteric coating agent,
wherein the weight ratio of the separating layer to the stabilizer is between 0.1 and 10, preferably between 0.1 and 5.0, more preferably between 0.5 and 2.0.

The separating layer constitutes a film coating layer.

The coating agents in the separating layer are selected from the group comprising hydroxypropylmethyl cellulose, amino alkyl methacrylate copolymers, polyethylene glycol (PEG), polyvinylpyrrolidone (PVP), polyvinyl alcohol, hydroxypropyl cellulose, lowsubstituted hydroxypropyl cellulose, sodium carboxymethyl cellulose, methyl and ethyl cellulose, hydroxyethyl methylcellulose, PVP(polyvinylpyrrolidone)/vinyl acetate copolymer, PVA (polyvinyl alcohol)/PEG copolymer, alginates, sugar, starch, sugar alcohols or mixtures thereof.

In a preferred embodiment, the coating agents are hydroxypropylmethyl cellulose, polyvinylpyrrollidone (PVP), polyethylene glycol (PEG) or mixtures thereof.

In one embodiment, the total coating agents in the separating layer and enteric coating agents in the enteric coated layer ranges from 11.0% to 55.0% by weight of the core.

According to one embodiment, the amount of dexlansoprazole or pharmaceutically acceptable salts or hydrated forms thereof is between 1.00 and 60.00% by weight of the total composition. Preferably this amount is between 5.00 and 40.00% by weight of the total composition. More preferably dexlansoprazole is present between 10.00 and 20.00% by weight of the total formulation.

According to these embodiments, the composition is in the form of coated tablet, trilayer tablet, bilayer tablet, multilayer tablet, mini tablet, pellet, sugar pellet, film-coated tablet, pill, capsule, oral granule, preferably a tablet.

The tablet dosage form can further be advantageous in that the manufacturing process can require fewer steps, e.g., eliminate the need for pellet formation and/or coating of those pellets, and there is no need for the additional expense of providing capsule shells.

It is known that capsules cost more than tablets and have significant space and potency limitations since their powdered contents cannot be compressed to a significant degree. Since capsules are not airtight, their shelf-life is shorter than tablets.

In one embodiment, the enteric coating agent in the enteric coated layer dissolves at pH 5.5 or higher and is selected from the group comprising cellulose acetate phthalate, cellulose acetate succinate, hydroxpropyl cellulose phthalate, hydroxpropyl ethylcellulose phthalate, hydroxyl propyl methyl cellulose phthalate, hydroxyl propyl methyl cellulose acetate succinate, hydroxyethyl cellulose phthalate, methylcellulose phthalate, polyvinyl acetate phthalate, polyvinylacetate hydrogen phthalate, amylase acetate phthalate, cellulose ester phthalates, cellulose ether phthalates, sodium cellulose acetate phthalate, starch acid phthalate, cellulose acetate butyrate, cellulose acetate maleate, cellulose acetate trimellitate, cellulose acetate propionate, styrene maleic acid dibutyl phthalate copolymer, styrene maleic acid polyvinyl acetate phthalate copolymer propionate, shellac or mixtures thereof.

Suitable stabilizers may comprise but not limited to alginic acid, aluminum hydroxide, aluminum oxide, calcium carbonate, calcium silicate, magnesium carbonate, magnesium oxide, magnesium trisilicate, potassium bicarbonate, sodium bicarbonate and sodium carbonate.

In a preferred embodiment, the stabilizer is present in an amount of between 5.00 and 20.00%, preferably between 5.00 and 15.00%, more preferably between 10.00 and 15.00% by weight of the total formulation.

According to one embodiment, the weight ratio of the stabilizer to dexlansoprazole or pharmaceutically acceptable salts or hydrated forms thereof is between 0.1 and 5.0, preferably between 0.1 and 2.0, more preferably between 0.5 and 1.0.

The ratio of the stabilizer to dexlansoprazole or pharmaceutically acceptable salts or hydrated forms thereof is effective to obtain a stable formulation. Especially magnesium oxide is preferred as stabilizer.

According to one embodiment, the composition comprises at least one pharmaceutically acceptable excipient selected from disintegrants, fillers, binders, lubricants, glidants, or mixtures thereof.

Suitable fillers may comprise but not limited to mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof.

In a preferred embodiment, the composition comprises at least two fillers which are fine mannitol and course mannitol.

Fine mannitol is the mannitol powder with the D50 value ranging between 1-180 µm, preferably lower than 60 µm. Preferably the fine mannitol is mannitol 60.

Coarse mannitol is the mannitol powder with the D50 value ranging between 20-600 µm, preferably lower than 400 µm. Preferably the coarse mannitol is mannitol DC 400.

The amount of fine mannitol is between 1-20%, preferably 5-15% and more preferably 5-10% by weight of the total composition.

The amount of coarse mannitol is between 1-20%, preferably 5-15% and more preferably 10-15% by weight of the total composition.

According to this preferred embodiment, the weight ratio of fine mannitol to course mannitol is between 0.1 and 5.0, preferably 0.2 and 2.0, more preferably 0.5 and 1.0. In this invention, the enteric coated compositions have improved stability, this preferred selection of range assures the enhanced stability during the shelf-life.

Suitable binders may comprise but not limited to microcrystalline cellulose, copovidone, copolyvidone, polyvinylpyrrolidone K30 (povidone K30), carnauba wax, pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), ethyl cellulose, polymetacrylates, polyethylene oxide, polyvinyl alcohol, polycarbophil, polyvinyl acetate and its copolymers, gelatin, xanthan gum, guar gum, alginate, carrageen, kollagen, agar, pectin, hyaluronic acid, carbomer, cellulose acetate phthalate, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

In a preferred embodiment, the binder is microcrystalline cellulose.

In one embodiment, the binder is present in an amount of between 10-30%, preferably 15-25%, more preferably 20-25% by weight of total formulation.

In one embodiment, the weight ratio of the binder to total coating agents which are in the separating layer and the enteric coated layer, is between 0.5 and 5.0, preferably between 1.0 and 2.0, more preferably between 1.5 and 2.0.

Suitable lubricants may comprise but not limited to magnesium stearate, glyceryl behenate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

In a preferred embodiment, the lubricant is magnesium stearate.

In one embodiment, the lubricant is present in an amount of between 0.1-5%, preferably between 0.5-5%, more preferably between 0.5-2% by weight of total formulation. Suitable glidants may comprise but not limited to colloidal silicon dioxide, talc, aluminium silicate, starch or mixtures thereof.

In a preferred embodiment, the glidant is colloidal silicon dioxide.

In one embodiment, the glidant is present in an amount of between 0.1-5, preferably between 0.1-2, more preferably between 0.5-1 by weight of total formulation.

Suitable disintegrants may comprise but not limited to sodium starch glycolate, croscarmellose sodium, sodium carbonate, hydroxylpropyl cellulose (HPC), cross-linked polyvinylpyrrolidone (crospovidone), copovidon, polycarbophil, low-substitue poloxamer, alginic acid and alginates, ion-exchange resins, magnesium aluminum silica, sodium carboxy methyl cellulose, carboxy methyl cellulose calcium, docusate sodium, guar gum, polyacrylin potasium, sodium alginate, sodium glycine carbonate or mixtures thereof.

In a preferred embodiment, the disintegrant is sodium starch glycolate.

In one embodiment, the disintegrant is present in an amount of between 1-20%, preferably between 5-15%, more preferably between 5-10% by weight of total formulation.

According to one preferred embodiment, the pharmaceutical composition comprises:
a. 1.00 to 60.00% dexlansoprazole or pharmaceutically acceptable salts or hydrated forms thereof
b. 5.00 to 20.00% magnesium oxide light
c. 1.00 to 20.00% sodium starch glycolate
d. 1.00 to 20.00% fine mannitol
e. 1.00 to 20.00% course mannitol
f. 10.00 to 30.00% microcrystalline cellulose
g. 0.10 to 5.00% magnesium stearate
h. 0.10 to 5.00% colloidal silicon dioxide
i. 1.00 to 20.00% separating layer
j. 1.00 to 20.00% enteric coating

Surprisingly it is found that, when the weight ratio of the enteric coating agent in the enteric coated layer to coating agents in the separating layer of the composition is in a specific amount, a synergistic effect is obtained over the release rate of the dexlansoprazole.

In this invention, the coated pharmaceutical compositions comprise:
a. a core comprising dexlansoprazole or pharmaceutically acceptable salts or hydrated forms thereof
b. a separating layer which has one or more coating agents,
c. an enteric coating layer which comprises at least one enteric coating agent,
wherein the weight ratio of enteric coating agent in the enteric coated layer to total coating agents in the separating layer is between 0.1 and 5.0, preferably between 0.5 and 3.0, more preferably between 1.0 and 1.5.

In one embodiment, the enteric coating agent in the enteric coated layer dissolves at pH 5.5 or higher and is selected from the group comprising cellulose acetate phthalate, cellulose acetate succinate, hydroxpropyl cellulose phthalate, hydroxpropyl ethylcellulose phthalate, hydroxyl propyl methyl cellulose phthalate, hydroxyl propyl methyl cellulose acetate succinate, hydroxyethyl cellulose phthalate, methylcellulose phthalate, polyvinyl acetate phthalate, polyvinylacetate hydrogen phthalate, amylase acetate phthalate, cellulose ester phthalates, cellulose ether phthalates, sodium cellulose acetate phthalate, starch acid phthalate, cellulose acetate butyrate, cellulose acetate maleate, cellulose acetate trimellitate, cellulose acetate propionate, styrene maleic acid dibutyl phthalate copolymer, styrene maleic acid polyvinyl acetate phthalate copolymer propionate, shellac or mixtures thereof.

The coating agents in the separating layer are selected from the group comprising hydroxypropylmethyl cellulose, amino alkyl methacrylate copolymers, polyethylene glycol (PEG), polyvinylpyrrolidone (PVP), polyvinyl alcohol, hydroxypropyl cellulose, lowsubstituted hydroxypropyl cellulose, sodium carboxymethyl cellulose, methyl and ethyl cellulose, hydroxyethyl methylcellulose, PVP(polyvinylpyrrolidone)/vinyl acetate copolymer, PVA (polyvinyl alcohol)/PEG copolymer, alginates, sugar, starch, sugar alcohols or mixtures thereof.

In a preferred embodiment, the coating agents are hydroxypropylmethyl cellulose, polyvinylpyrrollidone (PVP), polyethylene glycol (PEG) or mixtures thereof.

In one embodiment, the total coating agents in the separating layer and enteric coating agents in the enteric coated layer ranges from 11.0% to 55.0% by weight of the core.

In one embodiment, the core comprises one or more stabilizer.

Suitable stabilizers may comprise but not limited to alginic acid, aluminum hydroxide, aluminum oxide, calcium carbonate, calcium silicate, magnesium carbonate, magnesium oxide, magnesium trisilicate, potassium bicarbonate, sodium bicarbonate and sodium carbonate.

In a preferred embodiment, the stabilizer is present in an amount of between 5.00 and 20.00%, preferably between 5.00 and 15.00%, more preferably between 10.00 and 15.00% by weight of the total formulation.

According to one embodiment, the weight ratio of the stabilizer to dexlansoprazole or pharmaceutically acceptable salts or hydrated forms thereof is between 0.1 and 5.0, preferably between 0.1 and 2.0, more preferably between 0.5 and 1.0.

According to one embodiment, the amount of dexlansoprazole or pharmaceutically acceptable salts or hydrated forms thereof is between 1.00 and 60.00% by weight of the total composition. Preferably this amount is between 5.00 and 40.00% by weight of the total composition. More preferably dexlansoprazole is present between 10.00 and 20.00% by weight of the total formulation.

According to these embodiments, the composition is in the form of coated tablet, trilayer tablet, bilayer tablet, multilayer tablet, mini tablet, pellet, sugar pellet, film-coated tablet, pill, capsule, oral granule, preferably a tablet.

The tablet dosage form can further be advantageous in that the manufacturing process can require fewer steps, e.g., eliminate the need for pellet formation and/or coating of those pellets, and there is no need for the additional expense of providing capsule shells.

It is known that capsules cost more than tablets and have significant space and potency limitations since their powdered contents cannot be compressed to a significant degree. Since capsules are not airtight, their shelf-life is shorter than tablets

According to one embodiment, the composition comprises at least one pharmaceutically acceptable excipient selected from disintegrants, fillers, binders, lubricants, glidants, or mixtures thereof.

Suitable fillers may comprise but not limited to mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof.

In a preferred embodiment, the composition comprises at least two fillers which are fine mannitol and course mannitol.

Fine mannitol is the mannitol powder with the D50 value ranging between 1-180 µm, preferably lower than 60 µm. Preferably the fine mannitol is mannitol 60.

Coarse mannitol is the mannitol powder with the D50 value ranging between 20-600 µm, preferably lower than 400 µm. Preferably the coarse mannitol is mannitol DC 400.

The amount of fine mannitol is between 1-20%, preferably 5-15% and more preferably 5-10% by weight of the total composition.

The amount of coarse mannitol is between 1-20%, preferably 5-15% and more preferably 10-15% by weight of the total composition.

According to this preferred embodiment, the weight ratio of fine mannitol to course mannitol is between 0.1 and 5.0, preferably 0.2 and 2.0, more preferably 0.5 and 1.0. In this invention, the enteric coated compositions have improved stability, this preferred selection of range assures the enhanced stability during the shelf-life.

Suitable binders may comprise but not limited to polyvinylpyrrolidone (PVP, povidone), microcrystalline cellulose, copovidone, copolyvidone, polyvinylpyrrolidone K30 (povidone K30), carnauba wax, pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), ethyl cellulose, polymetacrylates, polyethylene oxide, polyvinyl alcohol, polycarbophil, polyvinyl acetate and its copolymers, gelatin, xanthan gum, guar gum, alginate, carrageen, kollagen, agar, pectin, hyaluronic acid, carbomer, cellulose acetate phthalate, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

In a preferred embodiment, the binder is microcrystalline cellulose.

In one embodiment, the binder is present in an amount of between 10-30%, preferably 15-25%, more preferably 20-25% by weight of total formulation.

In one embodiment, the weight ratio of the binder to total coating agents which are in the separating layer and the enteric coated layer, is between 0.5 and 5.0, preferably between 1.0 and 2.0, more preferably between 1.5 and 2.0.

Suitable lubricants may comprise but not limited to magnesium stearate, glyceryl behenate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

In a preferred embodiment, the lubricant is magnesium stearate.

In one embodiment, the lubricant is present in an amount of between 0.1-5%, preferably between 0.5-5%, more preferably between 0.5-2% by weight of total formulation.

Suitable glidants may comprise but not limited to colloidal silicon dioxide, talc, aluminium silicate, starch or mixtures thereof.

In a preferred embodiment, the glidant is colloidal silicon dioxide.

In one embodiment, the glidant is present in an amount of between 0.1-5, preferably between 0.1-2, more preferably between 0.5-1 by weight of total formulation.

Suitable disintegrants may comprise but not limited to sodium starch glycolate, croscarmellose sodium, sodium carbonate, hydroxylpropyl cellulose (HPC), cross-linked polyvinylpyrrolidone (crospovidone), copovidon, polycarbophil, low-substitue poloxamer, alginic acid and alginates, ion-exchange resins, magnesium aluminum silica, sodium carboxy methyl cellulose, carboxy methyl cellulose calcium, docusate sodium, guar gum, polyacrylin potasium, sodium alginate, sodium glycine carbonate or mixtures thereof.

In a preferred embodiment, the disintegrant is sodium starch glycolate.

In one embodiment, the disintegrant is present in an amount of between 1-20%, preferably between 5-15%, more preferably between 5-10% by weight of total formulation.

According to one preferred embodiment, the pharmaceutical composition comprises:
a. 1.00 to 60.00% dexlansoprazole or pharmaceutically acceptable salts or hydrated forms thereof
b. 5.00 to 20.00% magnesium oxide light
c. 1.00 to 20.00% sodium starch glycolate
d. 1.00 to 20.00% fine mannitol
e. 1.00 to 20.00% course mannitol
f. 10.00 to 30.00% microcrystalline cellulose
g. 0.10 to 5.00% magnesium stearate
h. 0.10 to 5.00% colloidal silicon dioxide
i. 1.00 to 20.00% separating layer
j. 1.00 to 20.00% enteric coating

### Example 1:

| **Ingredients** | **(%) amount (w/w)** |
|---|---|
| Dexlansoprazole or pharmaceutically acceptable salts or hydrated forms thereof | 10.00-20.00 |
| Magnesium oxide light | 10.00-15.00 |
| Sodium starch glycolate | 5.00-10.00 |
| Mannitol 60 (fine mannitol) | 5.00-10.00 |
| Mannitol DC 400 (course mannitol) | 10.00-15.00 |
| Microcrystalline cellulose PH 200 | 20.00-25.00 |
| Magnesium stearate | 0.50-2.00 |
| Colloidal silicon dioxide | 0.50-1.00 |
| **Separating layer** | **5.00-10.00** |
| **Enteric coating** | **5.00-10.00** |
| **Total coated tablet** | **100** |

### Example 2:

| **Ingredients** | **(%) amount (w/w)** |
|---|---|
| Dexlansoprazole or pharmaceutically acceptable salts or hydrated forms thereof | 1.00-60.00 |
| Magnesium oxide light | 5.00-20.00 |
| Sodium starch glycolate | 1.00-20.00 |
| Mannitol 60 (fine mannitol) | 1.00-20.00 |
| Mannitol DC 400 (course mannitol) | 1.00-20.00 |
| Microcrystalline cellulose PH 200 | 10.00-30.00 |
| Magnesium stearate | 0.10-5.00 |
| Colloidal silicon dioxide | 0.10-5.00 |
| **Separating layer** | **1.00-20.00** |
| **Enteric coating** | **1.00-20.00** |
| **Total coated tablet** | **100** |

### Example 3:

| **Ingredients** | **(%) amount (w/w)** |
|---|---|
| Dexlansoprazole or pharmaceutically acceptable salts or hydrated forms thereof | 16.64 |
| Magnesium oxide light | 11.09 |
| Sodium starch glycolate | 8.32 |
| Mannitol 60 (fine mannitol) | 8.32 |
| Mannitol DC 400 (course mannitol) | 12.48 |
| Microcrystalline cellulose PH 200 | 23.85 |
| Magnesium stearate | 1.66 |
| Colloidal silicon dioxide | 0.83 |
| **Separating layer** | **8.32** |
| **Enteric coating** | **8.49** |
| **Total coated tablet** | **100** |

The pharmaceutical compositions mentioned above are prepared by following these steps:
a. Weighging and mixing in a granulator dexlansoprazole or pharmaceutically acceptable salts or hydrated forms thereof, magnesium oxide light, fine mannitol, 30% of sodium starch glycolate and 35% od microcrystalline cellulose for 5 minutes.
b. Spraying water to the mixture and granulating it
c. Sieving the wet granules with a 5 mm sieving mesh
d. Drying the mixture in an oven at 45°C.
e. Sieving the dry granules with a 1 mm sieving mesh
f. Adding 70% of sodium starch glycolate, course mannitol, 65% of microcrystalline cellulose and colloidal silicon dioxide to the mixture and mixing 5 more minutes
g. Adding magnesium stearate to the mixture and mixing 2 more minutes
h. Compressing the powder mixture into tablets
i. Coating these tablets with a film coating to form a separating layer
j. Coating these coated tablets with an enteric coating

| **Separating layer ingredients** | | **(%) amount (w/w)** |
|---|---|---|
| Opadry Yellow | | 5.00% |
| HPMC 3 cP | 87.8% | |
| Propylene glycol | 8.7% | |
| PVP K-25 | 1.7% | |
| Titanium dioxide | 1.5% | |
| Yellow iron oxide | 0.1% | |
| Opadry White | | 95.00% |
| HPMC15cP | 72.8% | |
| Titanium dioxide | 14.0% | |
| Polyethylene glycol | 4.4% | |
| Magnesium stearate | 4.4% | |
| Glycerin | 4.4% | |

| **Enteric coating ingredients** | **(%) amount (w/w)** |
|---|---|
| AQOAT AS-LG (HPMC Acetyl Succinate) | 98.04% |
| Sodium hydroxide | 1.96% |

## Claims

1. An enteric coated pharmaceutical composition comprising;
a. a core comprising dexlansoprazole or a pharmaceutically acceptable salts or hydrated forms thereof, one or more stabilizer and at least one pharmaceutically acceptable excipient
b. a separating layer comprising one or more coating agents,
c. an enteric coating layer comprising at least one enteric coating agent, wherein the weight ratio of separating layer to stabilizer is between 0.1 and 10, preferably between 0.1 and 5.0, more preferably between 0.5 and 2.0.

2. The enteric coated pharmaceutical composition according to claim 1, wherein the total coating agents in the separating layer and enteric coated agents in the enteric coated layer ranges from 11.0% to 55.0% by weight of the core.

3. The enteric coated pharmaceutical composition according to claim 1, wherein the stabilizer is present in an amount of between 5.00 and 20.00%, preferably between 5.00 and 15.00%, more preferably between 10.00 and 15.00% by weight of the total formulation.

4. The enteric coated pharmaceutical composition according to claim 1 or 3, wherein the stabilizer is selected from a group comprising alginic acid, aluminum hydroxide, aluminum oxide, calcium carbonate, calcium silicate, magnesium carbonate, magnesium oxide, magnesium trisilicate, potassium bicarbonate, sodium bicarbonate and sodium carbonate; preferably the stabilizer is magnesium oxide.

5. The enteric coated pharmaceutical composition according to any preceding claims, wherein the weight ratio of the stabilizer to dexlansoprazole or pharmaceutically acceptable salts or hydrated forms thereof is between 0.1 and 5.0, preferably between 0.1 and 2.0, more preferably between 0.5 and 1.0.

6. An enteric coated pharmaceutical composition comprising;
a. a core comprising dexlansoprazole or pharmaceutically acceptable salts or hydrated forms thereof,
b. a separating layer comprising one or more coating agents,
c. an enteric coating comprising at least one enteric coating agent,
wherein the weight ratio of enteric coating agent in the enteric coated layer to total coating agents in the separating layer is between 0.1 and 5.0, preferably between 0.5 and 3.0, more preferably between 1.0 and 1.5.

7. The enteric coated pharmaceutical composition according to claim 1 or 6, wherein the enteric coating agent dissolves at pH 5.5 or higher and is selected from the group comprising cellulose acetate phthalate, cellulose acetate succinate, hydroxpropyl cellulose phthalate, hydroxpropyl ethylcellulose phthalate, hydroxyl propyl methyl cellulose phthalate, hydroxyl propyl methyl cellulose acetate succinate, hydroxyethyl cellulose phthalate, methylcellulose phthalate, polyvinyl acetate phthalate, polyvinylacetate hydrogen phthalate, amylase acetate phthalate, cellulose ester phthalates, cellulose ether phthalates, sodium cellulose acetate phthalate, starch acid phthalate, cellulose acetate butyrate, cellulose acetate maleate, cellulose acetate trimellitate, cellulose acetate propionate, styrene maleic acid dibutyl phthalate copolymer, styrene maleic acid polyvinyl acetate phthalate copolymer propionate, shellac or mixtures thereof, preferably hydroxyl propyl methyl cellulose acetate succinate.

8. The enteric coated pharmaceutical composition according to claim 1 or 6, wherein the one or more coating agent in the separating layer is selected from a group comprising hydroxypropylmethyl cellulose, amino alkyl methacrylate copolymers, polyethylene glycol (PEG), polyvinylpyrrolidone (PVP), polyvinyl alcohol, hydroxypropyl cellulose, lowsubstituted hydroxypropyl cellulose, sodium carboxymethyl cellulose, methyl and ethyl cellulose, hydroxyethyl methylcellulose, PVP(polyvinylpyrrolidone)/vinyl acetate copolymer, PVA (polyvinyl alcohol)/PEG copolymer, alginates, sugar, starch, sugar alcohols or mixtures thereof.

9. The enteric coated pharmaceutical composition according to claim 8, wherein the one more coating agent in the separating layer is preferably hydroxypropylmethyl cellulose, polyvinylpyrrollidone (PVP), polyethylene glycol (PEG) or mixtures thereof.

10. The enteric coated pharmaceutical composition according to claim 6, wherein the total coating agents in the separating layer and enteric coated agents in the enteric coated layer ranges from 11.0% to 55.0% by weight of the core.

11. The enteric coated pharmaceutical composition according to claim 6, wherein the core comprising one or more stabilizer.

12. The enteric coated pharmaceutical composition according to claim 11, wherein the stabilizer is selected from a group comprising alginic acid, aluminum hydroxide, aluminum oxide, calcium carbonate, calcium silicate, magnesium carbonate, magnesium oxide, magnesium trisilicate, potassium bicarbonate, sodium bicarbonate and sodium carbonate, preferably the stabilizer is magnesium oxide.

13. The enteric coated pharmaceutical composition according to claim 11 or 12, wherein the stabilizer is present in an amount of between 5.00 and 20.00%, preferably between 5.00 and 15.00%, more preferably between 10.00 and 15.00% by weight of total formulation.

14. The enteric coated pharmaceutical composition according to any preceding claim 11 to 13, wherein the weight ratio of the stabilizer to dexlansoprazole or pharmaceutically acceptable salts or hydrated forms thereof is between 0.1 and 5.0, preferably between 0.1 and 2.0, more preferably between 0.5 and 1.0.

15. The enteric coated pharmaceutical composition according to claim 1 or 6, wherein the composition comprises dexlansoprazole or pharmaceutically acceptable salts or hydrated forms thereof in an amount of between 1.00 and 60.00%, preferably between 5.00 and 40.00% and more preferably between 10.00 and 20.00% by weight of total formulation.

16. The enteric coated pharmaceutical composition according to claim 1 or 6, wherein said at least one pharmaceutically acceptable excipient is selected from disintegrants, fillers, binders, lubricants, glidants or mixtures thereof.

17. The enteric coated pharmaceutical composition according to claim 16, wherein the filler is selected from a group comprising mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof.

18. The enteric coated pharmaceutical composition according to claim 17, wherein the composition comprising at least two fillers which are fine mannitol and course mannitol.

19. The enteric coated pharmaceutical composition according to claim 18, wherein the weight ratio of fine mannitol to course mannitol is between 0.1 and 5.0, preferably 0.2 and 2.0, more preferably 0.5 and 1.0.

20. The enteric coated pharmaceutical composition according to any preceding claims, wherein the composition is in the form of coated tablet, trilayer tablet, bilayer tablet, multilayer tablet, mini tablet, pellet, sugar pellet, film-coated tablet, pill, capsule, oral granule, preferably in the form of a tablet.

21. The enteric coated pharmaceutical composition according to claim 16, wherein the binder is selected from a group comprising microcrystalline cellulose, copovidone, copolyvidone, carnauba wax, pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), ethyl cellulose, polymetacrylates, polyethylene oxide, polyvinyl alcohol, polycarbophil, polyvinyl acetate and its copolymers, gelatin, xanthan gum, guar gum, alginate, carrageen, kollagen, agar, pectin, hyaluronic acid, carbomer, cellulose acetate phthalate, hydroxyethyl methyl cellulose, polaxomer, polyethylene glycol (PEG), sugars, glycose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof, preferably microcrystalline cellulose.

22. The enteric coated pharmaceutical composition according to claim 21, wherein the weight ratio of the binder to total coating agents which are in the separating layer and the enteric coated layer, is between 0.5 and 5.0, preferably between 1.0 and 2.0, more preferably between 1.5 and 2.0.

23. The pharmaceutical composition according to any of the preceding claims, comprising:
a. 1.00 to 60.00% dexlansoprazole or pharmaceutically acceptable salts or hydrated forms thereof
b. 5.00 to 20.00% magnesium oxide light
c. 1.00 to 20.00% sodium starch glycolate
d. 1.00 to 20.00% fine mannitol
e. 1.00 to 20.00% coarse mannitol
f. 10.00 to 30.00% microcrystalline cellulose
g. 0.10 to 5.00% magnesium stearate
h. 0.10 to 5.00% colloidal silicon dioxide
i. 1.00 to 20.00% separating layer
j. 1.00 to 20.00% enteric coating

24. The process for preparation of the pharmaceutical composition according to claim 23, wherein the process comprising the following steps:
a. weighging and mixing dexlansoprazole or pharmaceutically acceptable salts or hydrated forms thereof, magnesium oxide light, fine mannitol, 30% of sodium starch glycolate and 35% of microcrystalline cellulose for 5 minutes in a granulator.
b. spraying water to the mixture and granulating it
c. sieving the wet granules
d. drying the mixture
e. sieving the dry granules
f. adding 70% of sodium starch glycolate, coarse mannitol, 65% of microcrystalline cellulose and colloidal silicon dioxide to the mixture and mixing 5 more minutes
g. adding magnesium stearate to the mixture and mixing 2 more minutes
h. compressing the powder mixture into tablets
i. coating these tablets with a film coating to form a separating layer
j. coating these coated tablets with an enteric coating
